# EUROPÄISCHE PATENTSCHRIFT

(11) **EP 1 844 722 B1**
(45) Veröffentlichungstag und Bekanntmachung des Hinweises auf die Patenterteilung: **20.04.2011**
(21) Anmeldenummer: 07014106.4
(22) Anmeldetag: 25.08.2003
(51) Int. Cl.: A61B 17/86

(54) **Knochenfixationseinrichtumg**
Fixing device for bone
Elément de retenue pour fixation osseuse

(30) Priorität: 04.10.2002 DE 10246386
(43) Veröffentlichungstag der Anmeldung: 17.10.2007
(62) Teilanmeldung aus: 03019180.3
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- WO-A-02/38054
- WO-A-88/03781
- US-A- 4 653 489
- US-A- 5 964 761
- US-A- 6 048 343
- US-A1- 2001 000 186
- KAISER W.; BURMESTER J.; LACHER V.: 'Zur Verfahrenswahl bei pertorchantären Femurfrakturen' UNFALLCHIRURGIE Bd. 25, Nr. 50-4, 1999,

## Beschreibung

Die vorliegende Erfindung bezieht sich auf eine Knochenfixationseinrichtung mit einer Knochenschraube mit einem Gewindeabschnitt mit einem netzartigen Aufbau.

Eine derartige Knochenschraube ist aus der DE 100 55 891 A1 bekannt. Sie beinhaltet einen Gewindeabschnitt, eine Spitze an dem einen Ende und einen Kopf zum Eingreifen mit einem Schraubendreher an dem anderen Ende. Der Gewindeabschnitt ist rohrförmig ausgebildet und seine Wandung weist eine Mehrzahl von Ausnehmungen auf.

Die bekannte Schraube kann z.B. bei der Behandlung von osteoporotischen Frakturen eingesetzt werden. Aufgrund der netzartigen Ausbildung der Schraube kann eine Fusion mit umgebendem Knochenmaterial stattfinden. Die Position der Fraktur im Knochen, die z.B. beim Hüftknochen oftmals weit von der Oberfläche entfernt ist, von der aus die Schraube eingeschraubt wird, erfordert zur sicheren Fixation eine präzise Positionierung der Knochenschraube und zum Teil auch eine mechanische Verbindung zu externen und/oder internen Verstrebungsimplantaten verbunden werden kann.

Aus der US 6,048,343 ist eine Knochenschraube mit einem rohrförmig ausgebildeten Gewindeabschnitt bekannt, wobei der rohrförmige Gewindeabschnitt auf seiner Außenwandung ein Knochengewinde aufweist und die Wandung des Gewindeabschnitts eine Mehrzahl von Ausnehmungen aufweist und wobei angrenzend an ein zweites Ende des Gewindeabschnitts ein mit diesem einstückig ausgebildeter knochengewindefreier Abschnitt vorgesehen ist.

Aus der US 4,653,489 ist eine Knochenfixationseinrichtung in Form einer hohlen, mit Öffnungen versehenen Hüftschraube zum Einbringen von Knochenzement in einen Knochenbereich mit Osteoporose bekannt.

Der vorliegenden Erfindung liegt die Aufgabe zugrunde, eine Knochenfixationseinrichtung mit verbesserten Eigenschaften und Einsatzmöglichkeiten bereitzustellen, die insbesondere präzise positionierbar ist.

Die Aufgabe wird gelöst durch eine Knochenfixationseinrichtung gemäß Anspruch 1. Weiterbildungen der Erfindung sind in dem Unteranspruch gekennzeichnet.

Die erfindungsgemäße Knochenfixationseinrichtung weist insbesondere den Vorteil auf, dass sie im Knochen an die erforderliche Stelle versenkbar ist. Somit erfüllt sie ihre Funktion als Zugelement, ohne dass ein Teil aus dem Knochen heraussteht.

Ferner lassen sich Knochenzement oder Wirkstoffe mittels der Knochenschraube präzise platzieren.

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine Explosionsdarstellung des modularen Aufbaus einer Knochenschraube gemäß einer ersten bis dritten Ausführungsform;
- Fig. 2: eine Darstellung der in Fig. 1 gezeigten Knochenschraube in zusammengesetztem Zustand;
- Fig. 3: eine Schrittfolge zum Befestigen einer äußeren Platte an einem Knochen.
- Fig. 4: eine Darstellung einer Kombination der Knochenschraube mit einem Marknagel.
- Fig. 5: eine Darstellung einer Knochenschraube gemäß einer vierten Ausführungsbeispiels;

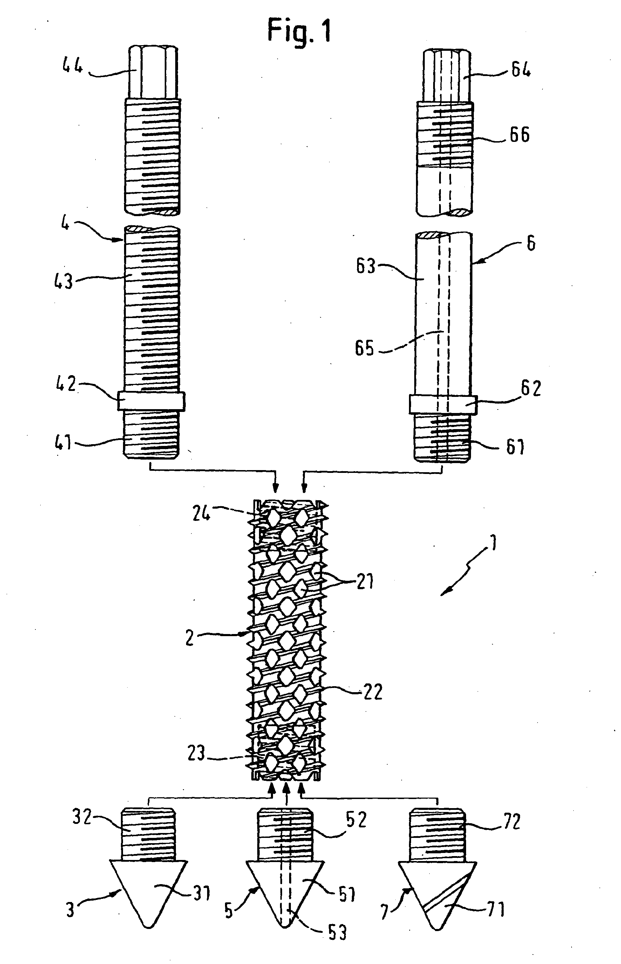

Die Figuren 1 bis 3 und 5 dienen der Erläuterung der Knochenschraub.

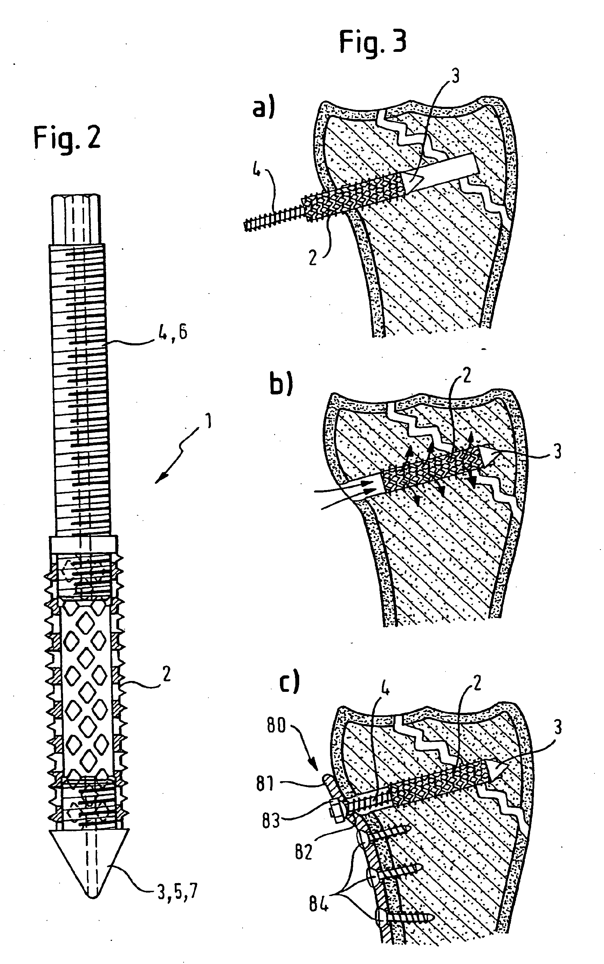

Im folgenden wird mit Bezug auf Fig. 1 und 2 eine Knochenschraube nach einem Beispiel beschrieben. Dabei ist in Fig. 2 zur Verbesserung der Übersichtlichkeit ein Teil der Knochenschraube im Schnitt dargestellt.

Ein erstes Beispiel der in Fig. 1 und 2 dargestellten modularen Knochenschraube 1 beinhaltet einen Gewindeabschnitt 2, eine Spitze 3 und ein Halteelement 4.

Der Gewindeabschnitt 2 ist rohrförmig ausgebildet und weist in seiner Wandung eine Vielzahl von Ausnehmungen 21 auf, die in dem gezeigten Ausführungsbeispiel rautenförmig ausgebildet sind. Die Rauten sind derart ausgerichtet, dass sich ihre Symmetrieachse jeweils parallel zu der Symmetrieachse des Rohres erstreckt. Die Rauten sind ferner gegeneinander versetzt angeordnet.

Auf der Außenwandung ist ein sogenanntes Knochengewinde 22 vorgesehen, welches in seiner Form den üblichen Knochen-schrauben entspricht.

Auf der Innenwandung weist der rohrförmige Gewindeabschnitt 2 an seinen beiden Enden je ein metrisches Innengewinde 23, 24 auf.

Die Spitze 3 beinhaltet den eigentlichen Spitzenteil 31 und einen Schaft 32. In dem gezeigten Ausführungsbeispiel weist der Schaft 32 ein Außengewinde auf, das dem Innengewinde 23 des rohrförmigen Gewindeabschnitts 2 entspricht, zum Verbinden der Spitze 3 mit dem Gewindeabschnitt 2 wie in Fig. 2 dargestellt.

Das Halteelement 4 weist an einem ersten Ende einen ersten Abschnitt 41 mit einem Außengewinde auf, das dem Innengewinde 23 des rohrförmigen Gewindeabschnitts 2 entspricht, zum Verbin-den des Halteelements 4 mit dem Gewindeabschnitt 2 wie in Fig. 2 dargestellt.

An dem Ende des Gewindeabschnitts 41 ist ein Anschlag 42 in Form einer Schulter bzw. eines ringförmigen Vorsprungs vorgesehen. Durch diesen Anschlag wird der Einschraubweg beim Einschrauben begrenzt. Wie insbesondere aus Fig. 2 ersichtlich, entspricht der Außendurchmesser des ringförmigen Vorsprungs 42 dem Außendurchmesser des rohrförmigen Gewindeabschnitts 2, so dass er beim Einschrauben der Knochenschraube in der durch diese geschaffenen Öffnung kein Hindernis darstellt.

Das Halteelement weist weiterhin einen stabförmigen Abschnitt 43 auf, der wie in Fig. 2 dargestellt in eingeschraubtem Zustand über das zweite Ende des Gewindeabschnitts 2 hinausragt. Der stabförmige Abschnitt 43 ist vorzugsweise durchgehend mit einem metrischen Außengewinde versehen.

Die axiale Länge des stabförmigen zweiten Abschnitts 43 ist bevorzugt größer als die des ersten, in den Gewindeabschnitt 2 einzuschraubenden Abschnitts 41. Sie ist in Abhängigkeit von dem Einsatzort der Knochenschraube so gewählt, dass der rohrförmige Gewindeabschnitt 2 an die erforderliche Stelle in dem Knochen versenkbar ist.

An dem seinem dem Abschnitt 41 gegenüberliegenden freien Ende weist das Halteelement ein Angriffselement für ein Einschraubwerkzeug in Form eines Außensechskantabschnitts 44 auf.

Der Gewindeabschnitt 2, die Spitze 3 und das Halteelement 4 sind vorzugsweise aus Titan geformt. Es kann jedoch auch ein anderes biokompatibles Material verwendet werden.

Im folgenden wird mit Bezug auf Fig. 2 und 3 der Einsatz der Knochenschraube nach dem ersten Beispiel beschrieben. Die Knochenschraube 1 wird dabei z.B. in das Ende eines Röhrenknochens eingesetzt, der durch Osteoporose von innen her geschwächt ist, z.B. in den Schenkelhals eines Oberschenkelknochens.

Zunächst wird die Spitze 3 in den rohrförmigen Gewindeabschnitt 2 eingeschraubt. Als nächstes wird das Halteelement 4 in den Gewindeabschnitt 2 eingeschraubt. Dadurch erhält man den in Fig. 2 dargestellten Aufbau.

Wie in Fig. 3 a) dargestellt, wird als nächstes die aus Gewindeabschnitt 2, Spitze 3 und Halteelement 4 zusammen-gesetzte Knochenschraube 1 in den Knochen hinein geschraubt. Dabei kann der Gewindeabschnitt 2 tief in den Knochen versenkt und dabei präzise an der Frakturstelle positioniert werden. Anschließend wird das Halteelement 4 wieder aus dem Gewindeabschnitt 2 herausgeschraubt.

Wie in Fig. 3 b) dargestellt, wird in einem weiteren Schritt in das Innere des Gewindeabschnitts 2 ein Füllmaterial wie z.B. Knochenzement oder Ähnliches und/oder ein medikamentöser oder wachstumsfördernder Wirkstoff eingespritzt. Im Gegensatz zu dem direkten Einspritzen sorgt der Gewindeabschnitt 2 für eine genauere Positionierung des Einspritzkanals und für eine gleichmäßigere Verteilung des eingespritzten Materials, das durch die Ausnehmungen 21 in die benachbarten Abschnitte des Knochens austreten kann. Dadurch wird die Fixierung des Knochens durch die Schraube weiter verbessert.

Die versenkte Knochenschraube dient als Verstärkung für die z.B. durch Osteoporose geschwächten Knochenbälkchen. Durch die Ausnehmungen 21 kann der Knochen in die Schraube einwachsen. Eine Stabilisierung der Frakturstelle des geschwächten Knochens erfolgt somit als Kombination von Zugentlastung und Fusion.

Fig. 3 c) zeigt eine Fixationseinrichtung 80 zum Fixieren des Knochens mit Hilfe einer äußeren Fixationsplatte. Dabei wird das Halteelement 4 nach dem Einspritzen des Füllmaterials wieder in den Gewindeabschnitt 2 eingeschraubt. Eine Platte 81 mit einer Ausnehmung 82 wird so an den Knochen angelegt, dass das freie Ende des Halteelements 4 durch die Ausnehmung 82 ragt, und durch Aufschrauben einer Mutter 83 auf das Außenge-winde des stabförmigen Abschnitts 43 des Halteelements 4 fest mit der Knochenschraube 1 verbunden. Weitere Knochenschrauben 84 dienen zum stabilen Fixieren der Platte 81 an dem Knochen. Der stabförmige Abschnitt 43 kann hierzu auf die erforderliche Länge gekürzt werden, die so bemessen ist, dass er nicht wesentlich über die Mutter 83 hervorsteht.

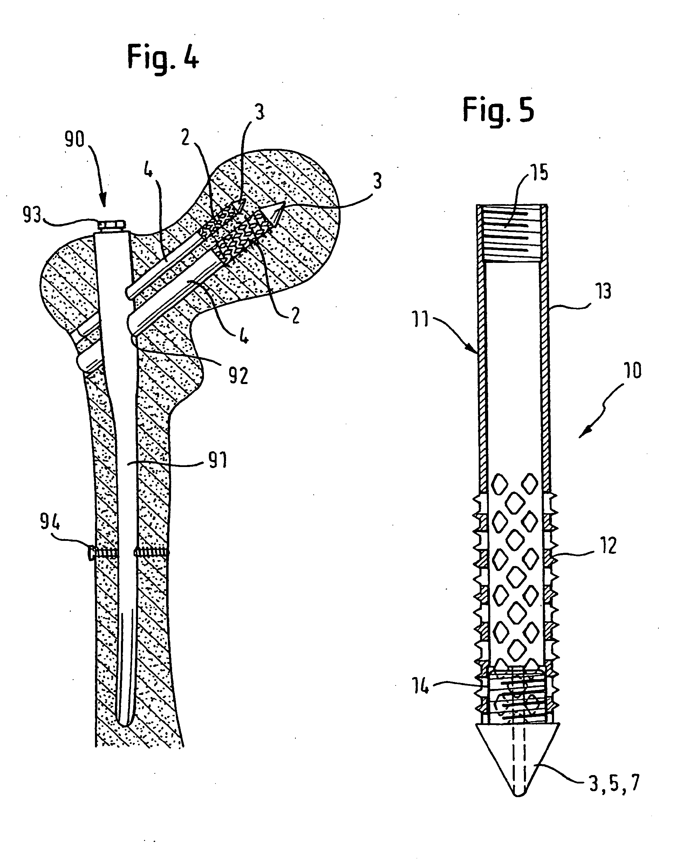

Fig. 4 zeigt ein Ausführungsbeispiel der Knochenfixationseinrichtung mit der Knochenschraube nach dem ersten Beispiel. Bei der Fixationseinrichtung 90 erfolgt die Fixierung des Knochens nicht Hilfe einer äußeren Fixationsplatte, sondern mit Hilfe eines in den Knochen eingebrachten Marknagels 91. Der Marknagel 91 weist zumindest eine Ausnehmung 92 auf, durch die zumindest ein Halteelement 4 einer Knochenschraube 1 hindurchragt. In dem dargestellten Ausführungsbeispiel sind es zwei Knochenschrauben 1 mit unterschiedlichen Durchmessern. Eine Verschlussschraube 93 verschließt den Marknagel nach außen, und eine Verriegelungsschraube (94) verhindert eine Bewegung in Längsrichtung.

Eine zweiten Beispiel der in Fig. 1 dargestellten modularen Knochenschraube 1 unterscheidet sich von den ersten Beispiel darin, dass anstelle der Spitze 3 eine kanulierte Spitze 5 und anstelle des Halteelements 4 ein kanuliertes Halteelement 6 vorgesehen ist.

Die kanulierte Spitze 5 unterscheidet sich von der Spitze 3 dadurch, dass zusätzlich eine koaxiale Bohrung 53 zum Hindurchleiten eines Wirkstoffs vorgesehen ist.

Das kanulierte Halteelement 6 unterscheidet sich von dem Halteelement 4 dadurch, dass zusätzlich eine koaxiale Bohrung 65 in vorgesehen ist und dass der stabförmige Abschnitt 63 nicht wie der stabförmige Abschnitt 43 bei dem ersten Beispiel über seine gesamte Länge mit einem metrischen Gewinde versehen ist, sondern nur in einem Teilabschnitt 66.

Alle anderen Merkmale stimmen mit dem ersten Beispiel überein, und auch der Einsatz erfolgt in der gleichen Weise.

Dieses Beispiel hat den Vorteil, dass sich auch ohne Herausschrauben des Halteelements und auch im Nachhinein z.B. mittels einer Spritze ein medikamentöser Wirkstoff präzise an die gewünschte Stelle einbringen lässt.

Ein drittes Beispiel der in Fig. 1 dargestellten modularen Knochenschraube 1 unterscheidet sich von dem ersten und zweiten Beispiel darin, dass anstelle der Spitze 3 eine selbstschneidende Spitze 7 vorgesehen ist. Alle anderen Merkmale stimmen mit dem ersten bzw. zweiten Beispiel überein, und auch der Einsatz erfolgt in der gleichen Weise.

Durch die selbstschneidende Spitze wird das Einbringen der Knochenschraube erleichtert.

Ein in Fig. 5 dargestelltes viertes Beispiel unterscheidet sich von dem in Fig. 1 und 2 dargestellten ersten bis dritten Beispiel in der Form des Gewindeabschnitts.

Die Knochenschraube 10 nach dem vierten Beispiel beinhaltet einen Schraubenabschnitt 11 und eine Spitze. Der Schraubenabschnitt 11 ist rohrförmig ausgebildet und besteht aus einem Gewindeabschnitt 12 und einem Abschnitt 13. Als Spitze kann jede der in dem ersten bis dritten Beispiel beschriebenen Spitzen 3, 5 und 7 verwendet werden.

Der Gewindeabschnitt 12 weist wie der bei dem ersten Beispiel beschriebene Gewindeabschnitt 2 in seiner Wandung eine Vielzahl von Ausnehmungen 21 und auf seiner Außenwandung ein Knochengewinde 22 auf.

Der Abschnitt 13 ist dagegen knochengewindefrei ausgebildet. In dem dargestellten Ausführungsbeispiel sind in dem knochengewindefreien Abschnitt 13 in der Wandung keine Ausnehmungen ausgebildet. Es können jedoch auch hier Ausnehmungen vorgesehen sein.

Auf der Innenwandung weist der rohrförmige Schraubenabschnitt 11 an seinen beiden Enden je ein metrisches Innengewinde 14, 15 auf.

Die axiale Länge des knochengewindefreien Abschnitts 13 ist in Abhängigkeit von dem Einsatzort der Knochenschraube so gewählt, dass der Gewindeabschnitt 12 an die erforderliche Stelle in dem Knochen versenkbar ist. Sie kann insbesondere auch größer sein als die axiale Länge des Gewindeabschnitts 12.

Der rohrförmige Schraubenabschnitt 11 ist vorzugsweise aus Titan geformt. Es kann jedoch auch ein anderes biokompatibles Material verwendet werden.

Auch der Einsatz des vierten Beispiels erfolgt in der gleichen Weise wie bei dem ersten bis dritten Beispiel. Zum Eindrehen der Knochenschraube 10 kann auf das Innengewinde 15 ein Halteelement 4, 6 aufgeschraubt werden, das später verbleibt. Das freie Ende des Abschnitts 13 ohne Knochengewinde kann auch selber ein Angriffselement für ein Einschraubwerkzeug aufweisen wie z.B. einen Kreuzschlitz.

Die beschriebenen Beispiele stellen lediglich Beispiele für die Ausführung der Erfindung dar. Gewindeabschnitt, Spitze und Halteelement können nicht nur wie in den Beispiele beschrieben miteinander kombiniert werden, sondern in beliebiger Weise. Dieser modulare Aufbau ermöglich eine Anpassung an die unterschiedlichsten Anforderungen.

Der Anschlag zum Begrenzen des Einschraubwegs des Halteelements 4, 6 in den Gewindeabschnitt 2 kann statt an dem Halteelement 4, 6 auch in dem rohrförmigen Gewindeabschnitt 2 vorgesehen sein, z.B. am Ende des Innengewindes 24.

Das Halteelement 4, 6 kann so ausgebildet sein, dass der stabförmige Abschnitt 43, 63 wie bei dem ersten Beispiel über seine ganze Länge ein Außengewinde aufweist oder wie bei dem zweiten Beispiel nur in einem Teilabschnitt. Der stabförmige Abschnitt 43, 63 kann aber auch gänzlich ohne Außengewinde ausgebildet sein. Der Durchmesser des stabförmigen Abschnitts 43, 63 kann wie in Fig. 1 und 2 dargestellt derselbe sein wie der des ersten Abschnitts 41, 61 des Halteelements 4, 6, er kann aber auch einen anderen, vorzugsweise kleineren Durchmesser aufweisen.

Auch der Halteabschnitt 14 nach dem vierten Beispiel kann über seine ganze Länge ein Außengewinde aufweisen oder wie bei dem zweiten Beispiel nur in einem Teilabschnitt.

Anstelle des Außensechskantabschnitts 44, 64 kann auch ein beliebiges anderes Angriffselement für ein Einschraubwerkzeug vorgesehen sein. So kann das freie Ende des Halteelement 4, 6 z.B. auch eine Ausnehmung für einen Inbusschlüssel aufweisen. Das Halteelement 4, 6 kann auch ohne Angriffselement 44, 64 für ein Einschraubwerkzeug ausgebildet sein. Das Einschrauben der Knochenschraube erfolgt dann mit einem geeigneten Werkzeug, das an dem stabförmigen Abschnitt 43 angreift.

Die Gewindeabschnitte 23, 24, 41, 43, 61, 66, 14 und 15 sind in den Beispiele als metrisch beschrieben. Alternativ dazu können die Gewinde auch zöllig oder in einer anderen geeigneten Form ausgebildet sein.

Anstelle der Innengewinde 23 und 24 bzw. 14 und 15 kann auch ein einziges Innengewinde ausgebildet sein, das sich über die gesamte Länge des Gewindeabschnitts 2 bzw. des Schraubenabschnitts 11 erstreckt. Das hat den Vorteil, dass das rohrförmige Gewindeabschnitt 2 auf jede beliebige Länge abgelängt werden kann, so dass Schrauben gewünschter Länge herstellbar sind und dadurch die Lagerhaltung wesentlich verringert werden kann.

Alternativ zu den rautenförmigen Ausnehmungen 21 können auch andere Öffnungsformen vorgesehen sein, insbesondere runde Öffnungen.

Die Spitze 3, 5, 7 kann nicht nur durch Einschrauben mit dem Gewindeabschnitt 2 bzw. 12 verbunden werden, sondern das erste Ende des Gewindeabschnitts 2 bzw. 12 und der Schaft 32, 52, 72 der Spitze 3, 5, 7 können ohne die jeweiligen Gewinde ausgebildet und in ihren Abmessungen so bestimmt sein, dass die Spitze 3, 5, 7 im Passsitz mit dem Gewindeabschnitt 2 bzw. 12 fest verbunden ist. Alternativ dazu kann die Spitze 3, 5, 7 auf eine beliebige andere Weise mit dem Gewindeabschnitt 2 bzw. 12 fest verbunden sein. Spitze und Gewindeabschnitt können auch einstückig ausgeführt sein.

Auch das Halteelement 4, 6 kann im Passsitz oder auf eine beliebige andere Weise mit dem Gewindeabschnitt 2 fest verbunden sein.

Alternativ zu der in Fig. 3 a) bis c) gezeigten Vorgehensweise kann der rohrförmige Gewindeabschnitt 2 vor dem Aufschrauben des Halteelements z.B. mit Knochenmaterial gefüllt werden. Anschließend wird die Schraube dann versenkt.

Die mit Bezug auf Fig. 3 beschriebene Kombination von Zugentlastung und Fusion kann natürlich nicht nur bei dem als Beispiel erwähnten Oberschenkelknochen durchgeführt werden, sondern bei beliebigen anderen Knochen, z.B. Tibia.

Die Knochenschraube 1, 10 ist nicht nur, wie in den Ausführungsbeispielen dargestellt, für das Einbringen in Röhrenknochen geeignet. Sie kann z.B. auch in Wirbeln oder anderen Knochen eingesetzt werden.

Das Halteelement 4, 6 kann nicht nur in Kombination mit dem rohrförmigen Gewindeabschnitt 2 verwendet werden, sondern auch mit einer beliebigen anderen Form von Knochenschrauben, die z.B. massiv oder kanuliert ausgebildet sein kann. Die Verbindung mit der Knochenschraube erfolgt in gleicher Weise wie die oben beschriebene Verbindung mit dem Gewindeabschnitt 2.

## Patentansprüche

1. Knochenfixationseinrichtung (90) mit
zumindest einer Knochenschraube (10) mit einem rohrförmig ausgebildeten Gewindeabschnitt (12) mit einer Spitze (3, 5, 7) an seinem ersten Ende und einem diesem gegenüberliegenden zweiten Ende,
wobei der rohrförmige Gewindeabschnitt (12) auf seiner Außenwandung ein Knochengewinde (22) aufweist und die Wandung des Gewindeabschnitts (12) eine Mehrzahl von Ausnehmungen (21) aufweist; und wobei
angrenzend an das zweite Ende des Gewindeabschnitts (12) ein mit diesem einstückig ausgebildeter knochengewindefreier Abschnitt (13) vorgesehen ist und
wobei an dem der Spitze gegenüberliegenden Ende des knochengewindefreien Abschnitts (13) ein Mittel zum Ein- oder Angreifen mit einem Einschraubwerkzeug und/oder ein Mittel (15) zum Befestigen eines Fixierelements vorgesehen ist;
**gekennzeichnet durch** ein Halteelement (4,6) mit einem ersten Abschnitt zum Verbinden mit dem der Spitze gegenüberliegenden Ende des knochengewindefreien Abschnitts (13) und einem stabförmigen zweiten Abschnitt (43,63) der in dem eingesetzten Zustand des Halteelements (4,6) über das der Spitze gegenüberliegende Ende des knochengewindefreien Abschnitts (13) hinausragt und
einen Marknagel (91) zum Fixieren eines Knochens mit einer Ausnehmung (92), **durch** die das freie Ende des stabförmigen Abschnitts des Halteelements zum Verbleib im implantierten Zustand hindurchgeführt ist.

2. Knochenfixationseinrichtung nach Anspruch 1, **dadurch gekennzeichnet, dass** die Länge des knochengewindefreien Abschnitts (13) gleich groß wie oder größer als die des Gewindeabschnitts (12) ist.

## Claims

1. Bone fixation device (90) having
at least one bone screw (10) having a tubular shaped thread section (12) with a tip (3, 5, 7) at the first end and a second end opposite to the first end,
wherein the tubular thread section (12) comprises a bone thread (22) on its outer wall and wherein the wall of the thread section (12) comprises a plurality of recesses (21); and wherein
adjacent to the second end of the thread section (12) a bone thread free section (13) integrally formed with the thread section (12) is provided and wherein
at the end of the bone thread free section (13) opposite to the tip, means for engaging in or engaging with the screw-in tool and/or means (15) for fixing a fixing element are provided, **characterized by**
a holding element (4, 6) having a first section for connecting with the end of the bone thread free section (13) opposite to the tip, and a rod shaped second section (43, 63) which projects beyond the end of the bone thread free section (13) opposite to the tip in the inserted state of the holding element (4, 6), and
an intramedullary nail (91) for fixing a bone having a recess (92) through which the free end of the tubular section of the holding element is guided for remaining in the implanted state.

2. Bone fixation device according to claim 1, **characterized in that** the length of the bone thread free section (13) is equal or greater as the length of the thread section (12).

## Revendications

1. Dispositif de fixation osseuse (90), avec :
au moins une vis à os (10), avec un tronçon fileté (12) tubulaire, avec une pointe (3, 5, 7) à sa première extrémité et une deuxième extrémité, opposée à celle-ci,
le tronçon fileté (12) tubulaire présentant, sur sa paroi extérieure, un filetage à os (22) et la paroi du tronçon fileté (12) tubulaire présentant une pluralité d'évidements (21), et où
en limite à la deuxième extrémité du tronçon fileté (12), est prévu un tronçon sans filetage à os (13), réalisé d'une seule pièce avec celui-ci,
sur l'extrémité, opposée à la pointe, du tronçon sans filetage à os (13), est prévu un moyen pour mettre en prise ou permettre la saisie avec un outil de vissage et/ou un moyen (15) pour fixer un élément de fixation ;
**caractérisé par** un élément de maintien (4, 6) avec un premier tronçon, pour relier à l'extrémité, opposée à la pointe, du tronçon sans filetage à os (13), et un deuxième tronçon (43, 63) en forme de tige qui, lorsque l'élément de maintien (4, 6) est à l'état monté, ressort de l'extrémité, opposée à la pointe, du tronçon sans filetage à os (13), et
un clou à moelle (91), pour la fixation d'un os, avec un évidemment (92), à travers lequel l'extrémité libre du tronçon en forme de tige de l'élément de maintien est passée, pour rester à l'état implanté.

2. Dispositif de fixation osseuse selon la revendication 1, **caractérisé en ce que** la longueur du tronçon sans filetage à os (13) est égale ou supérieure à celle du tronçon fileté (12).
